# EUROPÄISCHE PATENTANMELDUNG

(11) **EP 0 869 188 A2**
(43) Veröffentlichungstag der Anmeldung: **07.10.1998**
(21) Anmeldenummer: 98105294.7
(22) Anmeldetag: 24.03.1998
(51) Int. Cl.: C12Q 1/68

(54) **Verfahren zum Nachweis von Mikrosatelliten-Instabilität zur Tumordiagnostik**

(30) Priorität: 25.03.1997 DE 19712332
(71) Anmelder: BOEHRINGER MANNHEIM GMBH, 68298 Mannheim (DE)
(72) Erfinder: Dietmaier, Wolfgang, Dr., 93049 Regensburg (DE); Rüschoff, Josef, Prof., 93077 Bad Abbach (DE); Fishel, Richard, Prof., Penn Valley, PA 19072 (US)

(57) **Zusammenfassung**

Gegenstand der Erfindung ist ein Verfahren zur Bestimmung genomischer Instabilität an 5 ausgewählten Mikrosatelliten Loci. Die Analyse dieser ausgewählten Loci ist geeignet zur Erstellung von prognostischen Tumordiagnosen, zur Analyse von erblicher Tumorprädisposition sowie zur Tumorfrüherkennung. Besondere Bedeutung hat diese Methode bei der Diagnose von Tumoren des Gastrointestinaltraktes, beispielsweise Colorectaltumoren.

SEQUENZPROTOKOLL

(1) ALLGEMEINE ANGABEN:

(i) ANMELDER:
(A) NAME: Boehringer Mannheim GmbH
(B) STRASSE: Sandhoferstr. 116
(C) ORT: Mannheim
(E) LAND: DE
(F) POSTLEITZAHL: 68305
(G) TELEFON: 06217591456
(H) TELEFAX: 06217594457

(ii) BEZEICHNUNG DER ERFINDUNG: Verfahren zum Nachweis von
Mikrosatelliten-Instabilitaet zur Tumordiagnostik

(iii) ANZAHL DER SEQUENZEN: 50

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 1:

AAACAGGATG CCTGCCTTTA 20

(2) ANGABEN ZU SEQ ID NO: 2:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 2:

GGACTTTCCA CCTATGGGAC 20

(2) ANGABEN ZU SEQ ID NO: 3:

(i) SEQUENZKENNZEICHEN:
(A) LÄNGE: 22 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 3:

GGCAGTACCA CCTGTAGAAA TG 22

(2) ANGABEN ZU SEQ ID NO: 4:

(i) SEQUENZKENNZEICHEN:
(A) LÄNGE: 24 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 4:

GAGTAACAGA GGCATCGTGT ATTC 24

(2) ANGABEN ZU SEQ ID NO: 5:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 5:

ACTCACTCTA GTGATAAATC G 21

(2) ANGABEN ZU SEQ ID NO: 6:

(i) SEQUENZKENNZEICHEN:
(A) LÄNGE: 25 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 6:

AGCAGATAAG ACAGTATTAC TAGTT 25

(2) ANGABEN ZU SEQ ID NO: 7:

(i) SEQUENZKENNZEICHEN:
(A) LÄNGE: 25 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 7:

AGCTAAGTGA ACCTCATCTC TGTCT 25

(2) ANGABEN ZU SEQ ID NO: 8:

(i) SEQUENZKENNZEICHEN:
(A) LÄNGE: 24 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 8:

ACCCTAGCAC TGATGGTATA GTCT 24

(2) ANGABEN ZU SEQ ID NO: 9:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 9:

AACACTAGTG ACATTATTTT C 21

(2) ANGABEN ZU SEQ ID NO: 10:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 10:

AGCTAGGCCT GAAGGCTTCT 20

(2) ANGABEN ZU SEQ ID NO: 11:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 11:

ACCACTGCAC TTCAGGTGAC 20

(2) ANGABEN ZU SEQ ID NO: 12:

(i) SEQUENZKENNZEICHEN:
(A) LÄNGE: 22 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 12:

GTGATACTGT CCTCAGGTCT CC 22

(2) ANGABEN ZU SEQ ID NO: 13:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 13:

ATGACAAGCA ATCCTTGAGC 20

(2) ANGABEN ZU SEQ ID NO: 14:

(i) SEQUENZKENNZEICHEN:
(A) LÄNGE: 25 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 14:

CTGTGTTATA TCCCTAAAGT GGTGA 25

(2) ANGABEN ZU SEQ ID NO: 15:

(i) SEQUENZKENNZEICHEN:
(A) LÄNGE: 16 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 15:

CCCGTATGGC AACAGG 16

(2) ANGABEN ZU SEQ ID NO: 16:

(i) SEQUENZKENNZEICHEN:
(A) LÄNGE: 17 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 16:

TGTGCATGTC ATGAGTG 17

(2) ANGABEN ZU SEQ ID NO: 17:

(i) SEQUENZKENNZEICHEN:
(A) LÄNGE: 22 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 17:

TATTGGATAC TTGAATCTGC TG 22

(2) ANGABEN ZU SEQ ID NO: 18:

(i) SEQUENZKENNZEICHEN:
(A) LÄNGE: 21 Basenpaare
(B) ART: Nucleotid

(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 18:

TGCATCACCT CACATAGGTT A 21

(2) ANGABEN ZU SEQ ID NO: 19:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 19:

GGAAGAATCA AATAGACAAT 20

(2) ANGABEN ZU SEQ ID NO: 20:

(i) SEQUENZKENNZEICHEN:
(A) LÄNGE: 24 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 20:

GCTGGCCATA TATATATTTA AACC 24

(2) ANGABEN ZU SEQ ID NO: 21:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 21:

CAGGTTCTGT CATAGGACTA
20

(2) ANGABEN ZU SEQ ID NO: 22:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 22:

TTCTGGAAAC CTACTCCTGA 20

(2) ANGABEN ZU SEQ ID NO: 23:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 23:

CAGAAAATTC TCTCTGGCTA 20

(2) ANGABEN ZU SEQ ID NO: 24:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 24:

CTCATGTTCC TGGCAAGAAT 20

(2) ANGABEN ZU SEQ ID NO: 25:

(i) SEQUENZKENNZEICHEN:
(A) LÄNGE: 16 Basenpaare
(B) ART: Nucleotid
(C) STRANGFORM: Einzelstrang
(D) TOPOLOGIE: linear

(ii) ART DES MOLEKÜšLS: Genom-DNA

(xi) SEQUENZBESCHREIBUNG: SEQ ID NO: 25:

GCTCCCGGCT GGTTTT 16

(2) ANGABEN ZU SEQ ID NO: 26:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 26:

GCAGGAAATC GCAGGAACTT 20

(2) ANGABEN ZU SEQ ID NO: 27:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 27:

CTCTTTCTCT GACTCTGACC 20

(2) ANGABEN ZU SEQ ID NO: 28:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 28:

GACTTTCTAA GTTCTTGCCA G 21

(2) ANGABEN ZU SEQ ID NO: 29:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 29:

AGCGCAGCAC CTCCCGGCGC CAGTTT 26

(2) ANGABEN ZU SEQ ID NO: 30:

(i) SEQUENZKENNZEICHEN:
(A) LÄNGE: 27 Basenpaare
(B) ART: Nucleotid
(C) STRANGFORM: Einzelstrang
(D) TOPOLOGIE: linear

(ii) ART DES MOLEKÜšLS: Genom-DNA

(xi) SEQUENZBESCHREIBUNG: SEQ ID NO: 30:

GCTGCTGCTG CCTGGGGCTA GTCTCTT 27

(2) ANGABEN ZU SEQ ID NO: 31:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 31:

TCGCCTCCAA GAATGTAAGT 20

(2) ANGABEN ZU SEQ ID NO: 32:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜšLS: Genom-DNA

(xi) SEQUENZBESCHREIBUNG: SEQ ID NO: 32:

TCTGCATTTT AACTATGGCT C 21

(2) ANGABEN ZU SEQ ID NO: 33:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 33:

TGACTACTTT TGACTTCAGC C 21

(2) ANGABEN ZU SEQ ID NO: 34:

(i) SEQUENZKENNZEICHEN:
(A) LÄNGE: 22 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 34:

AACCATTCAA CATTTTTAAC CC 22

(2) ANGABEN ZU SEQ ID NO: 35:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 35:

ATTAACTTCC TACACCACAA C 21

(2) ANGABEN ZU SEQ ID NO: 36:

(i) SEQUENZKENNZEICHEN:
(A) LÄNGE: 19 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 36:

GTAGAGCAAG ACCACCTTG 19

(2) ANGABEN ZU SEQ ID NO: 37:

(i) SEQUENZKENNZEICHEN:
(A) LÄNGE: 29 Basenpaare

(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 37:

CGGTTATCCC AGTTCGGCCT CTCTGGGAT 29

(2) ANGABEN ZU SEQ ID NO: 38:

(i) SEQUENZKENNZEICHEN:
(A) LÄ[?]NGE: 28 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 38:

TCCACCTCCC GCTCAGTCAG ACTGCGCT 28

(2) ANGABEN ZU SEQ ID NO: 39:

(i) SEQUENZKENNZEICHEN:
(A) LÄNGE: 32 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 39:

GCAGCTATAA TGACTAGAAT GAAGTCCTAC TG 32

(2) ANGABEN ZU SEQ ID NO: 40:

(i) SEQUENZKENNZEICHEN:
(A) LÄNGE: 36 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 40:

TTGAATTAAA GACTTGTTTA AACACAAAAT TTAGAC 36

(2) ANGABEN ZU SEQ ID NO: 41:

(i) SEQUENZKENNZEICHEN:
(A) LÄNGE: 19 Basenpaare
(B) ART: Nucleotid
(C) STRANGFORM: Einzelstrang
(D) TOPOLOGIE: linear

(ii) ART DES MOLEKÜŠLS: Genom-DNA

(xi) SEQUENZBESCHREIBUNG: SEQ ID NO: 41:

TGGCGAGACT CCATCAAAG 19

(2) ANGABEN ZU SEQ ID NO: 42:

(i) SEQUENZKENNZEICHEN:
(A) LÄNGE: 22 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 42:

CTTTTTAAGC TGCAACAATT TC 22

(2) ANGABEN ZU SEQ ID NO: 43:

(i) SEQUENZKENNZEICHEN:
(A) LÄNGE: 19 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 43:

CTCCTCCCTA CTTACTTGT 19

(2) ANGABEN ZU SEQ ID NO: 44:

(i) SEQUENZKENNZEICHEN:
(A) LÄNGE: 19 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 44:

AATTAACAAG GTGTGGTGG 19

(2) ANGABEN ZU SEQ ID NO: 45:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 45:

GCACTTTCCT CAACTCTACA 20

(2) ANGABEN ZU SEQ ID NO: 46:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 46:

AACAGCTCCT TTAATGGCAG 20

(2) ANGABEN ZU SEQ ID NO: 47:

(i) SEQUENZKENNZEICHEN:
(A) LÄNGE: 24 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 47:

AGGGATACTA TTCAGCCCGA GGTG 24

(2) ANGABEN ZU SEQ ID NO: 48:

(i) SEQUENZKENNZEICHEN:
(A) LÄNGE: 22 Basenpaare

(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 48:

ACTGCCACTC CTTGCCCCAT TC 22

(2) ANGABEN ZU SEQ ID NO: 49:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 49:

CCCACAGCCT ATTCAGAACA C 21

(2) ANGABEN ZU SEQ ID NO: 50:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 50:

GTTGACTGCT GAACGGCTGC 20

## Beschreibung

Die Erfindung betrifft ein Verfahren und einen Kit zur prognostischen Diagnostik, Prädispositionsdiagnostik bzw. Früherkennung von Tumoren des Gastrointestinaltraktes, vorzugsweise Colorectaltumoren. Grundlage dafür bildet der Nachweis genomischer Instabilität von sogenannten Mikrosatelliten mit Hilfe von PCR.

Mikrosatelliten (MIS) sind kurze Tandem Repeats, die über das gesamte menschliche Genom verteilt vorkommen. Statistisch treten Mikrosatelliten etwa einmal in 100 000 Basenpaaren auf. Bisher sind 5 Klassen von MIS beschrieben, die sich nach der Länge ihrer kleinsten repetitiven Einheit als Mono-, Di-, Tri-, Tetra-, oder Pentanukleotid-Repeat voneinander unterscheiden. In der Regel treten diese repetitiven Einheiten 10 bis 40 mal in Tandemanordnung wiederholt auf. Mikrosatelliteninstabilität (MIN) in Form kleiner Deletionen oder Insertionen kann bei vielen Tumorpatienten nachgewiesen werden, wenn man DNA aus Tumormaterial mit normaler DNA des gleichen Individuums vergleicht (Thibodeau et al. (1993), Science, 260, 816-819) (WO 94/19492). Dies geschieht durch Amplifikation der DNA mit Hilfe von PCR und anschließender gelelektrophoretischer Auftrennung der Amplifikationsprodukte. Als Ursache für MIN wird ein dauerhafter Replikationsdefekt der Tumorzellen angesehen (Parsons et al., (1993), Cell, 75, 1227-1236; Shibata et al., (1994) Nat. Genet. 6, 273-281). Solche Tumoren werden als "Replikation-Error-Positive" (RER+) klassifiziert. Ein RER+ Phänotyp ist charakteristisch für Colorectaltumoren in Familien mit HNPCC (Hereditary Non-Polyposis Colon Cancer) (Aaltonen et al., (1993), Science, 260, 812-816).

Die Analyse von Mikrosatelliten ist eine äußerst attraktive Methode sowohl für diagnostische Anwendungen als auch für die Untersuchung der Tumorgenese von RER+ Tumoren. Aufgrund ihrer einfachen Durchführbarkeit ist die Bestimmung der MIN vor der Sequenzierung der Mismatch Repair Gene von HNPCC Familien ein geeignetes Hilfsmittel zur Identifizierung potentieller RER+ Patienten. Ebenfalls von großer Bedeutung ist die MIN Analyse als prognostische Diagnose bei sporadischem Colorectal-Karzinom, weil das Auftreten von MIN mit einer besseren Prognose korreliert (Lothe et al. (1993) Cancer Res., 53, 5849-5852; Thibodaeu et al. (1993), Science, 260, 816-819; Bubb et al. (1996) Oncogene, 12, 2641-2649).

MIN kann in mehr als 90% aller HNPCC Tumoren nachgewiesen werden (Liu et al., (1996) Nature Med., 2, 169-174), wohingegen MIN in sporadischen Colorectaltumoren nur mit einer Häufigkeit von 10-20% auftritt (Thibodeau et al. (1993) Science, 260, 816-819; Ionov et al. (1993), Nature, 363, 558-561; Aaltonen et al. (1993) Science, 260, 812-816; Lothe et al. (1993) Cancer Res., 53, 5849-5852). MIN ist jedoch nicht auf Colorectaltumoren beschränkt, sondern wurde auch in anderen Tumoren nachgewiesen. Dazu zählen unter anderem Pankreaskarzinome (Han et al. (1993) Cancer Res., 53, 5087-5089), gastrische Karzinome (Han et al. (1993) Cancer Res., 53, 5087-5089; Peltomaki et al. (1993) Cancer Res., 53, 5853-5855; Mironov et al. (1994) Cancer Res., 54, 41-44; Rhyu et al. (1994) Oncogene, 9, 29-32; Chong et al. (1994) Cancer Res., 54, 4595-4597), Prostata-Karzinome (Gao et al. (1994) Oncogene, 9, 2999-3003), Karzinome des Endometriums (Risinger et al. (1993) Cancer Res., 53, 5100-5103; Peltomaki et al. (1993) Cancer Res., 53, 5853-5855) und Mammakarzinome (Patel et al. (1994) Oncogene, 9, 3695-3700).

Der Mechanismus der Tumorgenese von RER+ Tumoren ist nicht im Detail bekannt. Bisher wurden fünf Gene identifizert, deren Defekt zu einem Auftreten des RER+ Phänotyps führen kann. Da in HNPCC Familien sowohl für hMLH1 (Bronner et al. (1994) Nature, 368, 258-261) als auch für hMSH2 (Fishel et al. (1993) Cell, 75, 1027-1038; Leach et al. (1993) Cell, 75, 1215-1225) genetische Variabilitäten mit einer Häufigkeit von über 30% nachgewiesen wurden, spielen diese beiden Gene offensichtlich eine wichtige Rolle bei der Ausprägung von MIN. 2 andere Mismatch Repair Gene, hPMS1 und hPMS2 sind in weniger als 5% aller HNPCC Patienten mutiert, so daß diese Gene wohl eine eher untergeordnete Rolle in RER+ Tumoren spielen. Es ist jedoch davon auszugehen, daß noch weitere, bisher unbekannte Gene an einem effektiven Mismatch Repair beteiligt sind.

Es besteht Grund zu der Annahme, daß MIN eine direkte Rolle bei der Tumorgenese dadurch spielt, daß aufgrund von Defekten im Mismatch Repair System Mikrosatelliten im kodierenden Bereich von Genen mutiert werden, die für die Regulation der Zellproliferation von Bedeutung sind. Beispielsweise wurde ein Repeat von 10 Deoxyadenosinen im kodierenden Bereich des TGFbeta1-Rezeptor Gens als MIN-Target identifiziert (Markowitz et al., (1995) Science, 268, 1336-1338). Ein weiteres MIN-Target, das IGF(II)R-Gen, ist in gastrointestinalen Tumoren innerhalb seiner kodierenden Region an einem (G)₈ Repeat mutiert (Souza et al. (1996) Nat. Genet., 14, 255-257). Interessanterweise waren nur 10% aller untersuchten Tumoren mit MIN in beiden Genen mutiert. Ein anderer (G)₈ MIS innerhalb eines Histon-Gens war in keinem der untersuchten Tumoren mutiert (Souza et al. (1996) Nat. Genet., 14, 255-257). Darüber hinaus konnte MIN bisher nur an einem Teil der untersuchten Loci nachgewiesen werden. Ob und inwieweit sich Mikrosatelliten, an denen Instabilität bereits nachgewiesen wurde, hinsichtlich der Häufigkeit des Auftretens von MIN unterscheiden, war zum Zeitpunkt der Erfindung nicht bekannt.

Vielmehr existieren für die Wahl von geeigneten Loci zur Analyse von MIN derzeit keine weiteren Anhaltspunkte. Es ist somit nicht bekannt, ob und wenn ja, welche Loci sich am besten zur eindeutigen Bestimmung von RER+ Phänotypen eignen. Stand der Technik hingegen ist die Analyse von 4 bis 7 zufällig ausgewählten Loci zur Klassifikation des MIN Status beispielsweise in Colorectalkarzinomen (z.B. Aaltonen et al. (1993) Science, 260, 812-816; Thibodeau et al. (1993) Science, 260, 816-819; Lothe et al. (1993) Cancer Res., 53, 5849-5852; Kim et al. (1994) Am. J. Path., 145, 148-156; Bubb et al. (1996) Oncogene, 12, 2641-2649; Plummer and Casey, (1996) Nat. Med., 2, 156-158).

Am häufigsten wurden dabei Mono- und Dinukleotid-Loci analysiert. Dinukleotid-Repeat Loci lassen sich in diesem Zusammenhang in 2 verschiedene Klassen einteilen:
- Nicht-komplexe Loci, welche innerhalb der zu amplifizierenden Region außer dem Dinukleotid Repeat keine weiteren repetitiven Elemente aufweisen (Klasse 2a). Zu diesen Loci gehören APC, D13S175; D3S1283, Mfd26, Mfd28 und Mfd 41.
- Komplexe Loci, bei denen neben dem Dinukleotid-Repeat noch weitere repetitive Sequenzen auftreten (Klasse 2b). Zu diesen Loci gehören Mfd15, D10S197, D11S1318, D11S904, D18S69, D2S123, D9S171 sowie TP53PCR.

Darüber hinaus wurden auch Mononukleotid-Repeat Loci aufgrund ihrer guten Amplifizierbarkeit sowie ihrer eindeutigen gelelektrophoretischen Signale mehrfach untersucht (Liu et al. (1996) Nature Med., 2, 169-174; Augenlicht et al. (1996) Oncogene, 12, 1767-1772; Plummer and Casey, (1996) Nat. Med., 2, 156-158).

Grundlage der Erfindung war somit die Suche nach polymorphen Loci, deren Analyse eine zuverlässige Aussage über die allgemeine Tendenz zur genomischen Instabilität zuläßt. Dabei konnten an unterschiedlichen Mikrosatelliten, an denen bereits nach dem Stand der Technik MIN gefunden wurde, unterschiedliche Häufigkeiten polymorpher Veränderungen nachgewiesen werden. Darüber hinaus konnte festgestellt werden, daß in unterschiedlichen Patienten verschiedene Klassen von Mikrosatelliten mit unterschiedlicher Häufigkeit von genomischer Instabilität betroffen sind. Daraus folgt, daß für eine zuverlässige Bestimmung des RER Phänotyps mit einer begrenzten Anzahl an PCR-Reaktionen eine Analyse verschiedener Klassen von MIS unabdingbar ist.

Gegenstand der Erfindung ist deshalb ein tumordiagnostisches Verfahren zur Analyse von Mikrosatelliten-Loci, bestehend aus folgenden Schritten:
a) Isolierung von genomischer DNA aus humanem biologischem Material
b) DNA-Amplifikation von 5 verschiedenen Mikrosatelliten-Loci mit Hilfe von jeweils fünf verschiedenen Primerpaaren, dadurch gekennzeichnet, daß es sich bei den zu amplifizierenden Loci um zwei Mononukleotid-Repeat Loci, ein oder zwei Dinukleotid-Repeat-Loci der Klasse 2a, ein oder zwei Dinukleotid-Repeat-Loci der Klasse 2b und 0 bis 1 Pentanukleotid-Repeat Locus handelt.
c) Größenbestimmung der Amplifikationsprodukte

Als vorteilhaft hat sich dabei insbesondere eine Ausführungsform erwiesen, bei der die 5 zu analysierenden Mikrosatelliten-Loci ausgewählt werden aus einer Gruppe von Loci bestehend aus: BAT 25, BAT26, BAT40, APC, Mfd15, D2S123, D18S69, und TP53Alu. Als besonders vorteilhaft hat sich eine spezielle Ausführungsform erwiesen, bei denen zur Analyse von 5 dieser Loci 5 Primerpaare aus einer Gruppe von Primern, repräsentiert durch SEQ ID NOs. 1, 2, 5, 6, 19, 20, 27, 28, 31, 32, 33, 34, 35, 36, 45 und 46, ausgewählt werden.

In einer speziellen Ausführungsform werden die 5 Loci BAT26, BAT40, APC, Mfd15 und D2S123 analysiert. Dabei können 1 oder mehrere Primerpaare entsprechend den SEQ ID NO. 1, 2, 5, 6, 19, 20, 33, 34, 35, 36 verwendet werden.

In einer weiteren speziellen Ausführungsform werden die 5 Loci BAT25, BAT26, APC, Mfd15 und D2S123 analysiert. Dabei können 1 oder mehrere Primerpaare entsprechend den SEQ ID NOs 1, 2, 5, 6, 19, 20, 31, 32, 33, 34 verwendet werden.

Gegenstand der Erfindung ist weiterhin die Anwendung dieses Verfahrens zur Bestimmung des RER Phänotyps, dadurch gekennzeichnet, daß bei fehlendem Nachweis von Mikrosatelliten-Instabilität bei allen 5 untersuchten Loci von einem RER- Phänotyp und bei Nachweis von Mikrosatelliten-Instabilität bei mehr als einem Locus von einem RER+ Phänotyp ausgegangen wird.

Eine besondere Ausführungsform besteht in der Anwendung des Verfahrens zur prognostischen Diagnose von Tumoren, vorzugsweise bei Tumoren des Endometriums, des Gastrointestinaltraktes und insbesondere bei Colorectaltumoren.

Eine andere besondere Ausführungsform der Erfindung besteht in der Anwendung des Verfahrens zur Diagnose einer familiären Tumor-Prädisposition, vorzugsweise für Tumoren des Endometriums, des Gastrointestinaltraktes und insbesondere für Colorectaltumoren.

Eine weitere besondere Ausführungsform der Erfindung besteht in der Anwendung des Verfahrens zur Früherkennung von Tumoren durch Nachweis von Mikrosatelliten-Instabilität in disseminierten Tumorzellen.

Eine zusätzliche Ausführungsform der Erfindung besteht in der Anwendung des Verfahrens vor einer Entscheidung, welche Art von Chemotherapie für einen Patienten eingesetzt werden soll. Dies ist von Bedeutung, da die Durchführung von Chemotherapien häufig mit unerwünschten Nebenwirkungen verbunden ist, so daß ein für bestimmte Arten von Tumoren unwirksamer Einsatz eines solchen Therapeutikums nach Möglichkeit zu vermeiden ist.

Gegenstand der Erfindung ist weiterhin ein Kit zur tumordiagnostischen Analyse von Mikrosatelliten-Instabilität, mindestens bestehend aus 5 Primer-Paaren, welche zur DNA-Amplifikation von zwei Mononukleotid-Repeat Loci, ein oder zwei Dinukleotid-Repeat-Loci der Klasse 2a, ein oder zwei Dinukleotid-Repeat-Loci der Klasse 2b und 0 bis 1 Pentanukleotid-Repeat Locus geeignet sind.

Eine besondere Ausführungsform des Kits beinhaltet mindestens 5 Primerpaare, die zur Amplifikation von 5 Loci, ausgewählt aus einer Gruppe bestehend aus BAT 25, BAT26, BAT40, APC, Mfd15, D2S123, D18S69 und TP53Alu., geeignet sind. Vorzugsweise besitzen diese Primer Sequenzen gemäß SEQ ID NO. 1, 2, 5, 6, 19, 20, 27, 28, 31, 32, 33, 34, 35, 36, 45 und 46.

Eine spezielle Ausführungsform des Kits beinhaltet mindestens 5 Primerpaare zur Analyse von BAT26, BAT40, APC, Mfd15, und D2S123 nachgewiesen. Dabei können 1 oder mehrere Primerpaare Sequenzen entsprechend den SEQ ID NO. 1, 2, 5 ,6 , 19, 20, 33, 34, 35, 36.... aufweisen.

Zusätzlich zu den 5 Primerpaaren können diese Kits weitere Primerpaare sowie molekularbiologische Reagenzien und Materialien enthalten, die der erfindungsgemäßen Durchführung von MIN Analysen dienen.

Als Quelle zur Gewinnung von genomischer DNA kann je nach Aufgabenstellung unterschiedliches biologisches Material analysiert werden. Für die prognostische Diagnostik sowie für die Diagnostik einer familiären Prädisposition wird dem Patienten entnommenes Tumorgewebe verwendet. Bei der Anwendung des erfindungsgemäßen Verfahrens zur Früherkennung von Tumoren durch Nachweis von MIN in disseminierten Tumorzellen wird die DNA aus zelluläre Bestandteile enthaltenden Körperflüssigkeiten oder Körperausscheidungen wie zum Beispiel Blut, Serum, Plasma, Urin oder Stuhl isoliert.

In der Regel wird eine Kontrollreaktion mit einer DNA durchgeführt, deren Sequenz dem "gesunden" Wild-Typ des zu analysierenden Mikrosatelliten-Locus entspricht. Besonders geeignet ist genomische DNA, die aus gesundem, nicht tumorigenem Gewebe desselben Individuums isoliert wurde.

Die Isolierung genomischer DNA aus Formalin-gefärbtem und in Paraffin eingebettetem Gewebe erfolgt folgendermaßen:
- Anfertigung von 5µm-Schnitten mit Mikrotom, Aufziehen auf einen Objektträger
- Deparaffinierung:

   Inkubation der Objektträger bei 65°C, 1 Stunde
   "Durchziehen" durch Alkoholreihe:
      2x 15 min in Xylol
      2x 15 min in EtOH(abs.)
      2x 15 min in EtOH (96%)
      2x 15 min in EtOH (70%)
         (mehrere Wochen in 70% EtOH haltbar)
- Überführen der Objektträger in Wasser
- Abkratzen des Gewebes im feuchten Zustand mit Skalpell, Glaskapillare, o.ä. (Mikrodissektion), überführen in 0,5 ml Reaktionsgefäß
- Zugabe von 20-50µl *Digestion-Buffer*:
   50 mM Tris-HCl, pH 7,5
   5% Tween20
   1 mM EDTA
- Zugabe von 7-15 µl ProteinaseK (20 mg/ml) (entspricht 30-50% des vorgegebenen Volumens)
- Inkubation bei 50°C im Thermoblock mit heizdeckel bis Lösung klar ist (über Nacht)
- Inaktivierung der Proteinase K: 15 min 94°C.

Fakultativ kann eine weitere Aufreinigung der DNA mit dem Quiagen tissue DNA Kit der Firma Quiagen erfolgen.

Zur Analyse des biologischen Materials wurden die PCR Ansätze nach folgendem Schema zusammenpipettiert:

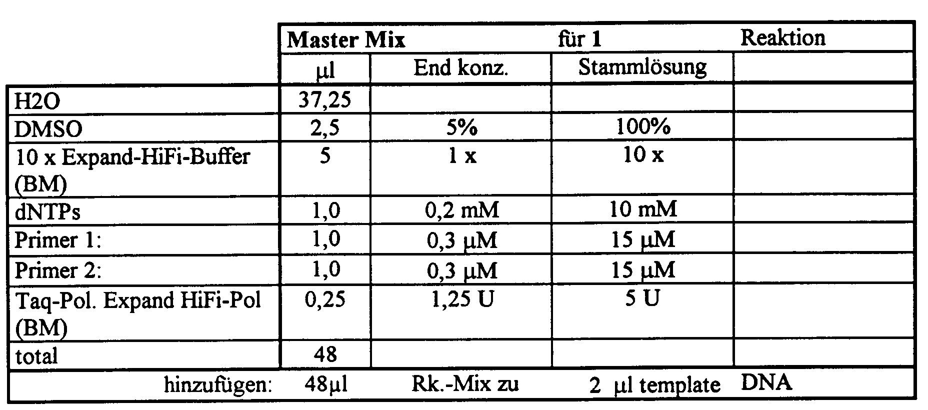

Alternativ wurden in einer Duplex- bzw. Multiplexanalyse auch 2 oder mehrere Loci in einem Reaktionsansatz zusammen analysiert, sofern deutlich voneinander unterscheidbare Fragmentgrößen zu erwarten waren. Dazu wurden 2 oder mehrere Primerpaare mit einer gleichen Endkonzentration von 0,3 µM je Primer eingesetzt.

Die PCR-Amplifikationen wurden unter Standardbedingungen mit 100 ng gereinigter genomischer DNA in einem MJ Research Thermocycler (PTC100, MJ Research, Watertown, MA) mit folgenden Zyklen durchgeführt:

| |
|---|
| 94°C 3 min (einmalige Denaturierung) |
| 35 Zyklen: |
| 94°C 1 min |
| Annealingtemperatur 50-68°C, 1 min |
| *entsprechend Abbildung 1* |
| 72°C 1 min |
| 72°C 8 min |

Anschließend wurden die PCR-Produkte aufeinem denaturierenden, 6,7%igen Polyacrylamidgel mit 50% Harnstoff für etwa eine Stunde bei 1800 Volt und 55°C in einer SequiGen Sequenzgelkammer (BioRad, Hercules, Ca) aufgetrennt und mit Silbernitrat (Budowle et al., (1991) Am. J. Hum. Genet., 48, 137-144) in einem modifizierten Färbebad (Bender et al., (1994) Biotechniques, 16, 204-206) angefärbt (Schlegl et al., (1995) Virchows Archiv, 426: 223-227).

### Polyacrylamidgelelektrophorese zur Auftrennung der PCR Banden: (6,7%tiges PA-6M Harnstoffgel, Vertikalapparatur sequi-GenGT, BioRad)

* 3µl PCR-Produkt
* 3 µl Loading buffer
   (10 ml Formamid
   10 mg Xylene Cyanol
   10 mg Bromphenolblau
   200 µl EDTA, 0,5M)
* Denaturierung, 94°C, 5 min.
* 15 min PA-Gelvorlauf bei 2300 V (bis 55°C erreicht ist)
* Beladen des PA-Gels
* 45-75 min Laufzeit bei 1800 V, 55°C

### Detektion der aufgetrennten PCR-Produkte durch Silberfärbung

* Wärmeaustauschplatte vom PA-Gel (zwischen Wärmeaustauschplatte und Glasplatte) abnehmen und Plexiglasfärberahmen auf das PA-Gel (an Glasplatte haftend) legen und mit Klammern fixieren.
* Zugabe von folgenden Lösungen:
   - H₂O: kurz spülen
   - 10% Ethanol: 10 min
   - 1% Salpetersäure: 3 min
   - H₂O: spülen
   - 0,012 M Silbernitrat: 20 min
   - H₂O: spülen
   - 0,28 M NaCO3/0,019% Formalin: spülen
   - 0,28 M NaCO3/0,019% Formalin: 3-6 min (bis Banden sichtbar)
   - 10% Essigsäure: 3 min
   - H₂O: 3 min
* Färberahmen entfernen
* Whatmann-3MM Papier auf PA-Gel legen und damit PA-Gel von Glasplatte abziehen
* PA-Gel mit Frischhaltefolie bedecken und 1 h im Geltrockner (GelDryingSystem iorad) trocknen (so behandelte Gele sind praktisch unbegrenzt haltbar)

DNA aus 27 Patienten mit Colorectalkarzinom wurde an 25 verschiedenen MIS-Loci auf MIN untersucht. Das ausgewählte Patientenkollektiv wurde aus einer Gruppe von 200 Patienten vorselektiert, bei denen in einer früheren prospektiven Studie 5 MIS Loci analysiert worden waren (APC, D9S171, TP 53, D13S175, D11S904). Bei diesen 27 Patienten war in 5 Fällen MIN an mindestens 2 Loci nachgewiesen worden, 5 weitere Fälle zeigten Instabilität an einem Locus und mußten daher als "lowMIN+" klassifiziert werden.
In 17 Fällen konnte keine Instabilität nachgewiesen werden. Zusätzlich wurden eine MIS-stabile Zelllinie (SW480) und eine Zellinie mit RER+Phänotyp (HCT116), welche einen Defekt im hMSH2 Mismatch Repair Gen aufweist, mit dem Tumormaterial verglichen.

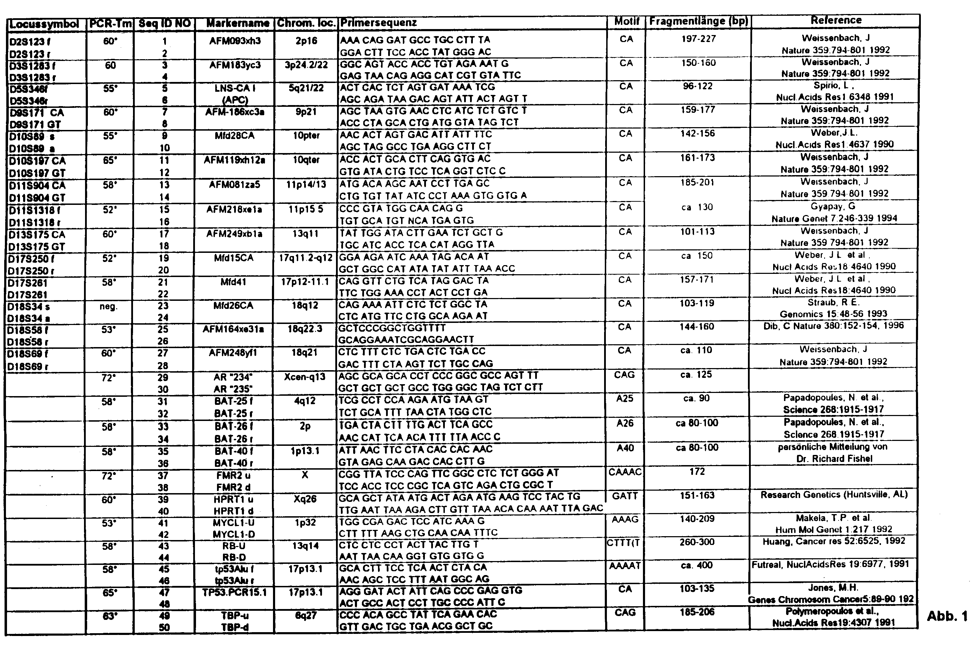

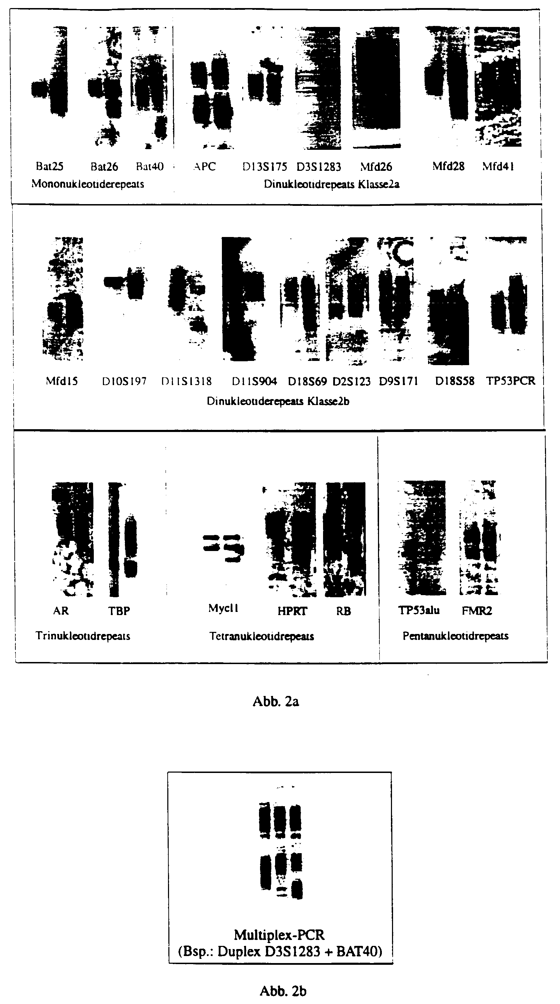

Für eine detailliertere Analyse des MIN-Status dieser Tumoren wurde die MIS-Analyse auf insgesamt 25 MIS Loci ausgedehnt. Vertreter aller sechs unterschiedlichen Repeat-Typen wurden analysiert: 3 Mononukleotid-Repeat Loci (BAT 25, BAT 26, BAT 40), 6 CA-Dinukleotid-Repeats der Klasse 2a (APC, D13S175, D3S1283, Mfd26, Mfd28 und Mfd41), 8 Dinukleotid-Repeats der Klasse 2b (Mfd15, D10S197, D11S1318, D11S904, D18S69, D2S123, D9S171, TP53PCR), zwei Loci mit Trinukleotid-Repeats (AR, TBP), drei Loci mit Tetranukleotid-Repeats (HPRT, MYCL1, RB), und zwei Loci mit Pentanukleotid-Repeats (FMR2, TP53alu). Die genauen Sequenzen dieser Repeats wurden entweder der GenBank Datenbank entnommen oder durch Direktsequenzierung von PCR-Produkten überprüft. Abbildung 1 gibt einen Überblick über die analysierten Loci sowie die jeweils zur Amplifikation verwendeten Primer, deren Sequenzen in SEQ ID NOs. 1-50 wiedergegeben sind. Abbildung 2a zeigt exemplarisch Amplifikationen unterschiedlicher Allele aller 25 untersuchten Genloci. Abbildung 2 b zeigt exemplarisch eine Duplexanalyse der Loci BAT40 und D3S1283. Bei Auftritt eines Verlustes von Allelen durch tumorbedingten Heterozygotizitäts-Verlust (loss of heterozygosity, LOH (LiMao et al., (1996), Science, 271, 659-662) wurde das Ergebnis der jeweiligen PCR in dieser Studie nicht mitberücksichtigt.

### Identifikation von RER+ Tumoren

Um abzuklären, welche Tumoren mit Sicherheit als RER+ klassifiziert werden können und um zu untersuchen, ob einige Tumoren als "schwach RER+" eingestuft werden müssen, wurden die verschiedenen Tumoren untereinander verglichen. Nach dem Stand der Technik existiert derzeit keine einfache Methode nach dem "entweder oder" Prinzip zur eindeutigen Klassifizierung eines Tumors als RER+ oder RER-. Das vorselektierte Kollektiv von 27 Colorectaltumorpatienten, von denen ursprünglich 17 als RER-, 5 als RER+ und 5 als "lowMIN+" diagnostiziert wurden, ergab nach Analyse weiterer Loci eine wesentlich differenziertes Bild bezüglich der Verteilung von MIN.

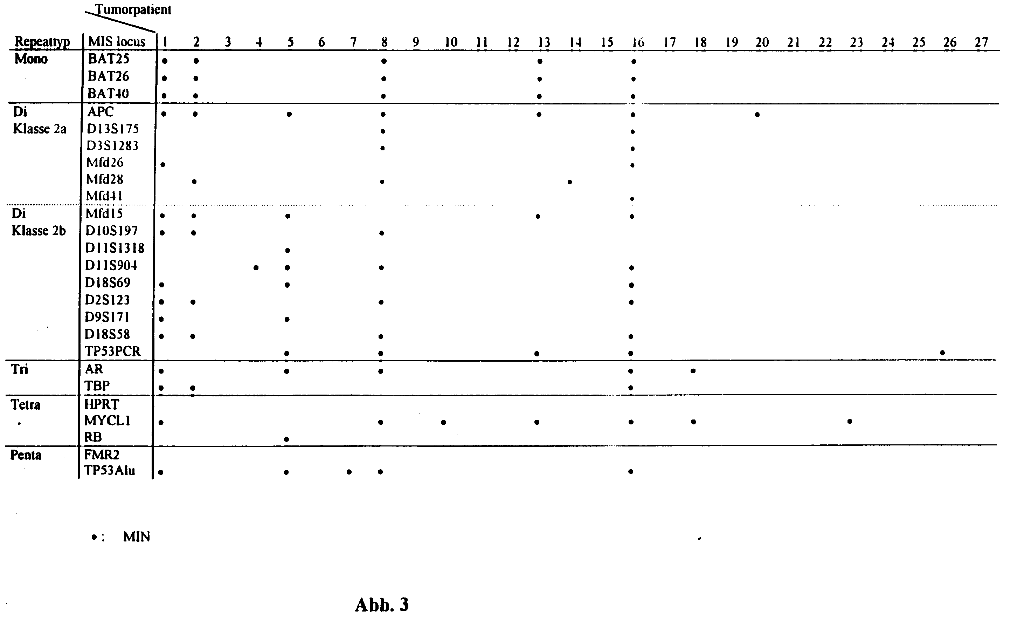

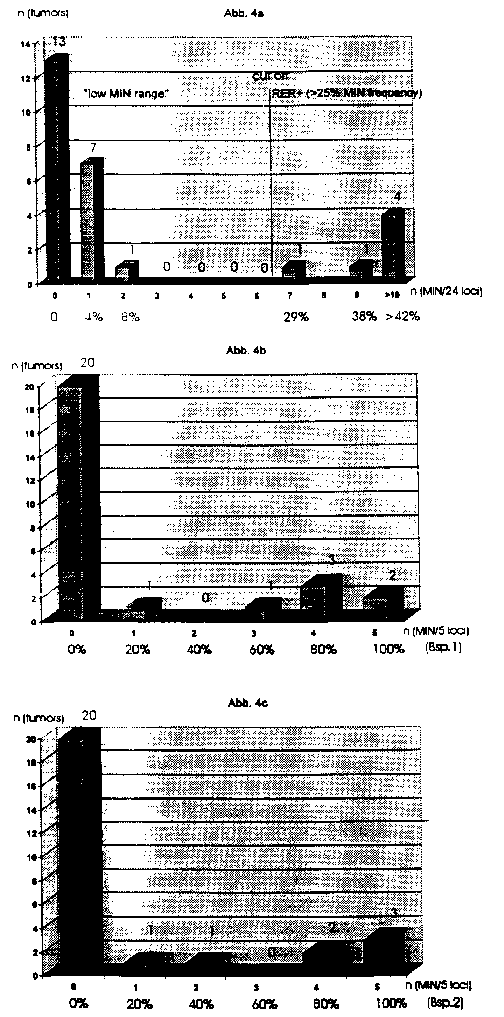

Wie in den Abbildungen 3 und 4a dargestellt, konnten 3 Tumoren mit einer MIN-Rate von mehr als 50% (14MIN/24 Loci, Nr. 1, 8 und 16), ein Tumor mit 42% (10MIN/24Loci, Nr. 5), ein Tumor mit 38% (9MIN/24Loci, Nr. 2) und ein Tumor mit 29% (7MIN/24Loci, Nr. 13) nachgewiesen werden. Damit besitzen all diese Tumoren als gemeinsames Kriterium eine Instabilitätsfrequenz von über 25% der analysierten Loci. Deshalb wurden diese insgesamt 6/27 Tumoren als eindeutig RER+ klassifiziert.

Darüber hinaus wurden 8 zusätzliche Tumoren identifiziert, die 1 bis 2 MIN-Ereignisse aufwiesen (n=8, MIN-Frequenz ≤ 8%) und daher als "lowMIN+" klassifiziert wurden. Im Vergleich zu der früheren Studie, bei der nur 5 anstatt 25 MIS Loci analysiert wurden, konnten durch die neue Studie nur 13 anstatt vorher 17 Fälle als RER- klassifiziert werden. Dieses Ergebnis, erzielt durch eine Ausweitung der Analyse auf 25 MIS-Loci, unterscheidet sich damit grundlegend von früheren Studien nach dem Stand der Technik und verdeutlicht das Problem einer unzuverlässigen RER-Klassifikation, wenn für eine solche Klassifikation eine kleine Anzahl an MIS Loci zufällig ausgewählt wird. In diesem Zusammenhang ist allerdings von besonderer Bedeutung, daß kein Tumor mit einer mittleren Instabilität an 3 bis 6 Loci entsprechend einem Prozentsatz von 10-25% nachgewiesen werden konnte, so daß bei einer MIN Rate von über 25% eine RER+ Klassifikation eindeutig vorgenommen werden kann.

### Unterschiedliche MIS Loci zeigen unterschiedliche MIN-Häufigkeiten

Insgesamt waren in 14 der 27 untersuchten Tumoren Mikrosatelliten von Instabilität betroffen. Wie erwartet, trat MIN dabei in einigen Tumoren vermehrt auf; zusätzlich wurden jedoch auch einzelne Ereignisse an MIN in weiteren Tumoren nachgewiesen. Um zu ermitteln, ob MIN-Häufigkeit vom Repeat-Typus abhängt, wurden die MIN-Frequenzen für jeden Repeat-Typ separat ermittelt und mit der durchschnittlichen MIN-Frequenz der Gesamtheit der getesteten Mikrosatelliten verglichen: Die durchschnittliche MIN-Rate bezogen auf alle pro Patient untersuchten Loci betrug 11,4% (78 MINs/25Loci=3,1 MIN/Locus; durchschnittliche MIN Rate = 3,1/27 Patienten = 11,4%). Die durchschnittlichen Frequenzen innerhalb der einzelnen Repeat-Typen waren dagegen unterschiedlich: sämtliche Mononukleotid-Repeats waren überdurchschnittlich oft verändert (5,0 MINs/27 Patienten = 18,5%= + 7,1%); alle anderen Repeat Typen waren seltener als die Mononukleotid Repeats betroffen. Sowohl die MIN Raten von komplexen Dinukleotid-Loci als auch von nicht komplexen Dinukleotid Loci unterschieden sich nicht signifikant von dem für die Gesamtheit aller Loci bestimmten Mittelwert (0,3 bzw. 0,9%). Ähnliches gilt für die Tetranukleotid-Repeats (+1,4%), die allerdings bezogen auf den jeweils einzelnen Locus eine starke Heterogenität aufweisen (-11,4% bis + 14,5%). Erhöhte MIN Frequenz wurde für beide Trinukleotid Repeats ermittelt (+3,4%). Pentanukleotid Repeats zeigten dagegen unterdurchschnittliche MIN Frequenzen (-4,0%). An einem Locus dieses Typs (FMR2) wurde überhaupt keine MIN nachgewiesen. Daraus folgt, daß die Bestimmung der Frequenz von MIN-Ereignissen dramatisch von der Auswahl der analysierten Loci abhängig ist.

### Bestimmte MIS Loci sind häufiger spezisch in RER+ Tumoren verändert

Deshalb ist für die Analyse des MIN Status von Bedeutung, ob es bestimmte Loci gibt, die spezifisch und regelmäßig in RER+ Tumoren von MIN betroffen sind. Ein einheitliches Ergebnis wurde diesbezüglich nur bei MIS mit Mononukleotid Repeats erzielt (BAT 25, BAT 26, BAT 40). Jeder dieser Loci war in den gleichen 5 RER+ Tumoren verändert (Nrs. 1, 2, 8, 13, 16), aber keiner wies MIN in RER- Tumoren oder "lowMin" Tumoren auf. Im Gegensatz dazu waren bis auf Mfd15 alle anderen getesteten Loci entweder weniger oft in den RER+ Tumoren mutiert oder zusätzlich in "lowMIN" Tumoren verändert. Beispielsweise konnte für den APC Locus nicht nur in allen RER+ Tumoren, sondern auch in Tumor Nr. 20 MIS nachgewiesen werden. Fünf Loci zeigten MINs in 4/6 RER+ Tumoren, aber nur D2S123 war in Nicht-RER+ Tumoren unverändert. Im Gegensatz dazu zeigte Locus MYCL1, der ebenfalls in 4/6 RER+ Tumoren verändert war, zusätzlich Instabilitäten in 3 "lowMIN" Tumoren, so daß beispielsweise dieser Locus als Marker ungeeignet ist.

Daher erfordert eine Beschränkung auf 5 Marker zur Analyse von Mikrosatelliteninstabilität eine gezielte Auswahl der Loci, so daß dennoch gewährleistet ist, daß die Zahl der nicht eindeutig zu klassifizierenden lowMIN+ Fälle minimiert wird und alle RER+ Träger mit einem höchst möglichen Maß an Wahrscheinlichkeit identifiziert werden können.

### Kurze Beschreibung der Abbildungen

Abbildung 1 zeigt eine Tabelle mit den wichtigsten Kennzeichen der analysierten MIS Loci (Locussymbol, Markername, chromosomale Lokalisation, Repeat-Typ) sowie den Parametern für die jeweilige PCR-Amplifikation (PCR-Tm: Hybridisierungstemperatur).

Abbildung 2a zeigt die gelelektrophoretische Analyse von 25 untersuchten Mikrosatelliten Loci. Die verschiedenen Allele der erfindungsgemäß zu analysierenden Loci BAT26, BAT40, APC, MfD15, D2S123 und TP53Alu lassen sich deutlich voneinander unterscheiden. Abbildung 2b zeigt exemplarisch eine Duplexanalyse der Loci BAT40 und D3S1283 in einem Reaktionsansatz.

Abbildung 3 stellt das Ergebnis der durchgeführten Studie in einer Übersicht dar. 27 Patienten mit Colorectaltumoren wurden auf MIN an 25 verschiedenen Allelen untersucht. Das Ergebnis zeigt, daß (i) unterschiedliche MIS in unterschiedlicher Häufigkeit von polymorphen Veränderungen betroffen sind und (ii) in unterschiedlichen Patienten verschiedene Klassen von Mikrosatelliten mit unterschiedlicher Häufigkeit von genomischer Instabilität betroffen sind.

Abbildung 4 klassifiziert die Tumoren aus dem untersuchten Patientenkollektiv von 27 Personen nach der Anzähl der ermittelten MIN Ereignisse.
Abbildung 4a repräsentiert die Auswertung aller 25 MIS. Die Verteilung zeigt, daß eine Gruppe von 6 Patienten existiert, bei denen MIN häufiger als 7 mal auftritt, so daß dieser Klasse eindeutig der Phänotyp RER+ zugeordnet werden kann. Darüber hinaus existiert eine Gruppe von 8 Patienten, bei denen 1 oder 2 MIN nachgewiesen wurden und die damit als "lowMIN+" zu klassifizieren sind.
Abbildung 4b repräsentiert die erfindungsgemäße Analyse von 5 ausgewählten MIS wie in Beispiel 1 beschrieben. Diese Analyse führt ebenfalls zu einer Verteilung, aufgrund derer eine eindeutige Entscheidung über den RER+ Phänotyps getroffen werden kann. Nur 2 Patienten (Nr. 7, TP53 Alu Locus und Nr. 20, APC Locus) müssen nach diesem Verfahren als low MIN+ klassifiziert werden.
Abbildung 4c repräsentiert die Analyse einer anderen erfindungsgemäßen Auswahl von 5 MIS gemäß Beispiel 2, die mit einer Ausnahme (APC, Patient 20) ebenfalls eine eindeutige Klassifikation des RER Phänotyps ermöglicht.

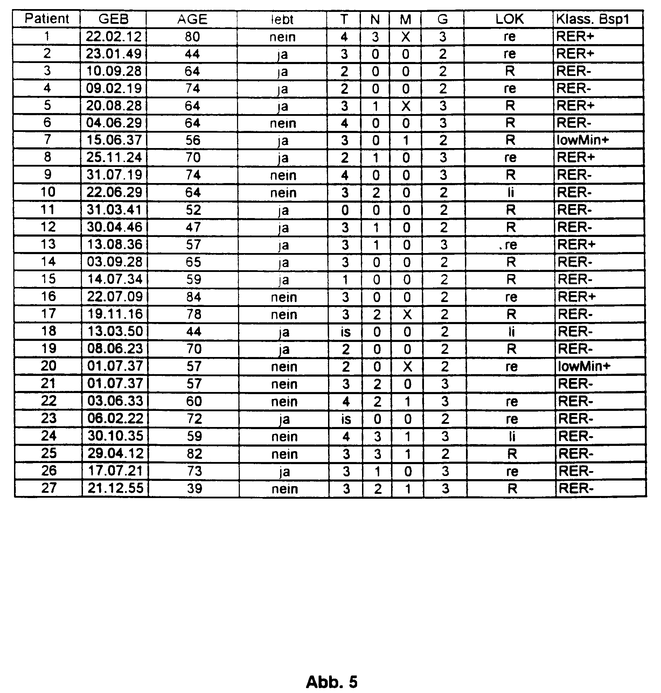

Abbildung 5 zeigt Geburtsdatum, Alter und klinische Daten zum untersuchten Patientenkollektiv, Stand August 1994. (T, N, M: Tumorklassifikation, G: Grade, LOK: Tumorlokalisation, re: Colon rechts, li: Colon links, R: Rectum).

Die folgenden Beispiele erläutern die Erfindung weiter:

### Beispiel 1: Auswahl von 2 Mononukleotid-Repeat Loci, 1 Dinukleotid-Repeat-Locus der Klasse 2a, 1 Dinukleotid Locus der Klasse 2b und 1 Pentanukleotid-Repeat Locus

Wie die Studie zeigte, ergaben sich bei der Verwendung von Mononukleotid Repeat Loci zur Bestimmung RER+ Phänotyps keine falsch-positiven Resultate, so daß eine Analyse dieser Loci besonders geeignet erschien. Andererseits konnten nicht alle RER+ Tumoren durch die Analyse von Mononukleotid Repeat Loci nachgewiesen werden. So war beispielsweise Tumor Nr. 5 eindeutig RER+, wies aber keine Instabilität bezüglich der Loci BAT25, BAT26 und BAT40 auf, obwohl MIN an 9 Loci mit anderen Repeat Typen nachgewiesen werden konnten. Es ergab sich daher die Notwendigkeit, für eine möglichst exakte Bestimmung des RER Phänotyps bei einer begrenzten Anzahl von 5 analysierten Loci eine Kombination aus verschiedenen Repeat Typen auszuwählen. Dadurch wird gewährleistet, daß trotz der geringen Zahl an analysierten Loci einerseits alle RER+ Träger mit einem höchst möglichen Maß an Wahrscheinlichkeit identifiziert werden können und andererseits die Zahl der nicht eindeutig zu Klassifizierenden lowMIN+ Fälle soweit wie möglich minimiert wird.

Aufgrund der durch die Studie mit 27 Tumoren ermittelten Häufigkeiten wurden zur Bestimmung des Phänotyps folgende Kriterien festgelegt: bei mindestens 2 MIS-positiven Loci sollte von einem RER+ Phänotyp ausgegangen werden, bei keinem positiven Nachweis von MIS sollte von einem RER- Phänotyp ausgegangen werden. Der Nachweis von genau einem MIN Ereignis wurde als "low MIN+" definiert. (Letzteres erfordert im Zweifelsfalle die Analyse weiterer MIS Loci.)

Die Auswertung der 27 Tumoren bezüglich der erfindungsgemäß ausgewählten Loci BAT 26, BAT 40, APC, Mfd15, TP53Alu nach diesem Verfahren führte zu dem in Abbildung 4b dargestellten Ergebnis. Für alle 6 Tumoren, die durch die Analyse von 25 Loci als RER+ klassifiziert worden waren, wurde eine Häufigkeit von mindestens drei MIN Ereignissen bestimmt. Somit wurden diese Tumoren auch durch die Analyse der 5 ausgewählten Loci als RER+ klassifiziert. Nur zwei Tumoren (Nr. 7, Nr. 20) wurden nach diesem Verfahren als "lowMIN+" klassifiziert und sind somit nicht eindeutig zu interpretieren.

### Beispiel 2: Auswahl von 2 Mononukleotid-Repeat Loci, 1 Dinukleotid-Repeat-Locus der Klasse 2a und 2 Dinukleotid Loci der Klasse 2b

Die Auswertung der 27 Tumoren erfolgte nach dem gleichen Verfahren wie in Beispiel 1, jedoch mit einer modifizierten, ebenfalls erfindungsgemäßen MIS-Auswahl (BAT26, BAT 40, APC, Mfd15 und D18S69). Das Ergebnis ist in Abbildung 4c dargestellt. Sämtliche Tumoren mit RER+ Phänotyp waren in mindestens drei der ausgewählten Loci verändert. Somit konnten auch durch diese Auswahl an Loci alle 6 bekannten RER+ Tumoren eindeutig als RER+ identifiziert werden. Nur ein Tumor wurden in diesem Falle als "lowMIN+ und damit als nicht eindeutig interpretierbar klassifiziert.

### Beispiel 3: Bestimmung des RER Phänotyps als prognostischer Indikator

Zum Nachweis der Eignung einer erfindungsgemäßen Auswahl von 5 Loci zur Bestimmung des RER Phänotyps wurden die in Abbildung 5 tabellarisch enthaltenen klinischen Daten mit den Ergebnissen der MIN Analyse aus Beispiel 1 verglichen. Wie in diesem Beispiel offenbart, führte die Analyse der getesteten Loci zum Nachweis von RER+ bei 6 von insgesamt 27 untersuchten Tumorpatienten. Nur 2 von 6 (33%) dieser RER+ Patienten waren zum Abschluß der Studie verstorben. Beide wären zu diesem Zeitpunkt über 80 Jahre alt. Im Gegensatz dazu waren bereits 8 von 19 (42%) der in Beispiel 1 als RER- klassifizierten Patienten verstorben. Ihr Altersduchschnitt hätte zum Zeitpunkt der Studie 64 Jahre betragen. Daraus ist ersichtlich, daß eine erfindungsgemäße Analyse von 5 Mikrosatelliten Loci eine prognostische Aussage über den Verlauf der Tumorerkrankung ermöglicht.

## Patentansprüche

1. Verfahren zur Analyse von Mikrosatelliten-Loci, bestehend aus folgenden Schritten:
a) Isolierung von genomischer DNA aus humanem biologischem Material;
b) DNA-Amplifikation von 5 verschiedenen Microsatelliten-Loci der DNA mit Hilfe von jeweils fünfverschiedenen Primerpaaren, wobei es sich bei den zu amplifizierenden Loci um zwei Mononukleotid-Repeat Loci, ein oder zwei Dinukleotid-Repeat-Loci der Klasse 2a, ein oder zwei Dinukleotid-Repeat-Loci der Klasse 2b und gegebenenfalls ein Pentanukleotid-Repeat Locus handelt;
c) Größenbestimmung der Amplifikationsprodukte.

2. Verfahren gemäß Anspruch 1, dadurch gekennzeichnet, daß die 5 Mikrosatelliten-Loci ausgewählt werden aus einer Gruppe von Loci bestehend aus: BAT 25, BAT26, BAT40, APC, MfD15, D2S123, D18S69 und TP53Alu.

3. Verfahren gemäß Anspruch 2, dadurch gekennzeichnet, daß mindestens 1 Primerpaar ausgewählt wird aus einer Gruppe von Primern repräsentiert durch SEQ ID NOs. 1, 2, 5, 6, 19, 20, 27, 28, 31, 32, 33, 34, 35, 36, 45 und 46.

4. Verfahren gemäß Anspruch 2, dadurch gekennzeichnet, daß die 5 Mikrosatelliten-Loci: BAT26, BAT40, APC, Mfd15 und D2S123 analysiert werden.

5. Verfahren gemaß Anspruch 3, dadurch gekennzeichnet, daß mindestens 1 Primerpaar ausgewählt wird aus einer Gruppe von Primern repräsentiert durch SEQ ID NOs. 1, 2, 5, 6, 19, 20, 33, 34, 35, und 36.

6. Verfahren gemäß Anspruch 2, dadurch gekennzeichnet, daß die 5 Mikrosatelliten Loci BAT25, BAT26, APC, Mfd15 und D2S123 analysiert werden.

7. Verfahren gemaß Anspruch 3, dadurch gekennzeichnet, daß mindestens ein Primerpaar ausgewählt wird aus einer Gruppe von Primers repräsentiert durch SEQ ID NOs 1, 2, 5, 6, 19, 20, 31, 32, 33 und 34.

8. Verfahren gemaß einem der Ansprüche 1-7 zur Bestimmung des RER Phänotyps, dadurch gekennzeichnet, daß bei fehlendem Nachweis von Mikrosatelliten-Instabilität bei allen 5 untersuchten Loci von einem RER- Phänotyp und bei Nachweis von Mikrosatelliten-Instabilität bei mehr als einem Locus von einem RER+ Phänotyp ausgegangen wird.

9. Verwendung eines Verfahrens gemäß einem der Ansprüche 1-8 zur prognostischen Tumordiagnose.

10. Verwendung eines Verfahrens gemäß einem der Ansprüche 1-8 zur Diagnose von familiärer Tumor-Prädisposition.

11. Verwendung gemäß Anspruch 9 oder 10 zur Indikation von Tumoren des Gastrointestinalsystems und des Endometriums.

12. Verwendung gemaß Anspruch 11 zur Indikation von Colorectalkarzinomen.

13. Verwendung gemäß einem der Ansprüche 1-8 zur Früherkennung von Tumoren durch Nachweis von Mikrosatelliten Instabilität in disseminierten Tumorzellen.

14. Verwendung gemaß einem der Ansprüche 1-8 zur Therapieentscheidung.

15. Kit zur tumordiagnostischen Analyse von Mikrosatelliten-Instabilität, mindestens bestehend aus 5 Primerpaaren , welche zur DNA-Amplifikation von zwei Mononukleotid-Repeat Loci, ein oder zwei Dinukleotid-Repeat-Loci der Klasse 2a, ein oder zwei Dinukleotid-Repeat-Loci der Klasse 2b und gegebenenfalls einem Pentanukleotid-Repeat Locus geeignet sind.

16. Kit zur tumordiagnostischen Analyse von Mikrosatelliten-Instabilität, bestehend aus 5 Primerpaaren, welche zur DNA-Amplifikation von 5 Loci, ausgewählt aus einer Gruppe bestehend aus BAT 25, BAT26, BAT40, APC, MfD15, D2S123, D18S69 und TP53Alu, geeignet sind.

17. Kit zur tumordiagnostischen Analyse von Mikrosatelliten-Instabilität, bestehend aus 5 Primerpaaren, von denen mindestens 1 Primerpaar ausgewählt wird aus einer Gruppe von Primern repräsentiert durch SEQ ID NOs. 1, 2, 5, 6, 19, 20, 27, 28, 31, 32, 33, 34, 35, 36, 45 und 46.

18. Kit gemäß Anspruch 15, dadurch gekennzeichnet, daß 5 Primerpaare enthalten sind, welche zur DNA-Amplifikation von BAT26, BAT40, APC, Mfd15, und D2S123 geeignet sind.

19. Kit gemäß Anspruch 16, dadurch gekennzeichnet, mindestens 1 Primerpaar enthalten ist, welches ausgewählt wird aus einer Gruppe von Primern repräsentiert durch SEQ ID NOs. 1, 2, 5, 6, 19, 20, 33, 34, 35, und 36.

20. Kit gemaß Anspruch 15, dadurch gekennzeichnet, daß 5 Primerpaare enthalten sind, welche zur DNA Amplifikation von BAT25, BAT26, APC, Mfd15 und D2S123 geeignet sind.

21. Kit gemäß Anspruch 16, dadurch gekennzeichnet, daß mindestens 1 Primerpaar enthalten ist, welches ausgewählt wird aus einer Gruppe von Primern repräsentiert durch SEQ ID NOs 1, 2, 5, 6, 19, 20, 31, 32, 33 und 34.
